(19) Europäisches Patentamt
European Patent Office
Office européen des brevets

(11) Veröffentlichungsnummer: 0 442 385 A2

# (12) EUROPÄISCHE PATENTANMELDUNG

(21) Anmeldenummer: 91101731.7

(22) Anmeldetag: 08.02.91

(51) Int. Cl.[5]: C07D 501/18, C07D 501/26, A61K 31/545

(30) Priorität: 13.02.90 DE 4004370

(43) Veröffentlichungstag der Anmeldung: 21.08.91 Patentblatt 91/34

(84) Benannte Vertragsstaaten: AT BE CH DE DK ES FR GB GR IT LI LU NL SE

(71) Anmelder: HOECHST AKTIENGESELLSCHAFT
Postfach 80 03 20
W-6230 Frankfurt am Main 80(DE)

(72) Erfinder: Adam, Friedhelm, Dr.
Rheingaustrasse 46
W-6238 Hofheim am Taunus(DE)
Erfinder: Dürckheimer, Walter, Dr.
Im Lerchenfeld 45
W-6234 Hattersheim am Main(DE)
Erfinder: Hörlein, Rolf, Dr.
Assmannshäuser Weg 21
W-6000 Frankfurt am Main(DE)
Erfinder: Mencke, Burghard, Dr.
Hauptstrasse 2
W-5409 Holzappel(DE)

## (54) Verfahren zur Herstellung von 7-Amino-3-methoxymethyl-ceph-3-em-4-carbonsäure.

(57) Verfahren zur Herstellung der Verbindung der Formel

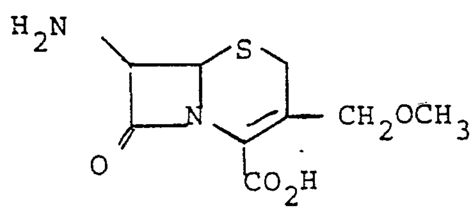

durch Umsetzung einer Verbindung der Formel

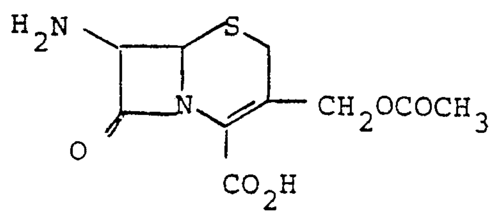

oder eines geeigneten Salzes derselben mit einem Gemisch aus Methanol, Methansulfonsäure und Trifluormethansulfonsäure.

EP 0 442 385 A2

## VERFAHREN ZUR HERSTELLUNG VON 7-AMINO-3-METHOXYMETHYL-CEPH-3-EM-4-CARBONSÄURE

7-Amino-3-alkoxymethyl-ceph-3-em-4-carbonsäuren sind wertvolle Ausgangsmaterialien für die Herstellung von oral applizierbaren Cephalosporinen durch anschließende Acylierung der Aminogruppe, wie z.B. solchen, die in EP-B-134 420 und EP-A-329 008 beschrieben sind.

Die bislang bekannt gewordenen Austauschverfahren zur Herstellung von Verbindungen der allgemeinen Formel A

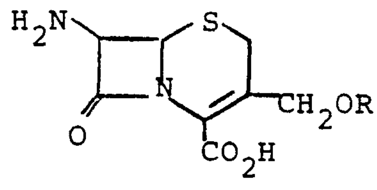

(A)

in der R einen Alkylrest bedeutet, zeichnen sich durch Reaktionsbedingungen aus, die eine Herstellung im technischen Maßstab erheblich einschränken. So wird z.B. in EP-A-262 744 ein Verfahren beschrieben, nach dem Verbindungen der Formel A durch Umsetzung des entsprechenden Alkohols mit einem großen Überschuß an anorganischen Lewissäure-Katalysatoren und nachfolgender Chromatographie an Ionenaustauschern erhalten werden. Ein solches Verfahren ist technisch und ökologisch nicht effizient durchführbar. In der EP-A-204 657 werden Derivate der Formel A unter Verwendung von technisch schwer handhabbarem, gasförmigem Bortrifluorid und unter Verwendung von Lösungsmitteln hergestellt. Die so gewonnenen Derivate müssen in der Regel weiter gereinigt werden.

Ein weiteres, in der japanischen Anmeldung JP 59/163 387 beschriebenes Verfahren benutzt starke organische Säuren als Katalysatoren und chlorierte Kohlenwasserstoffe als Lösungsvermittler. Eine Nacharbeitung des beschriebenen Verfahrens lieferte die gewünschten Produkte in geringer Ausbeute (ca. 30 %) und Reinheit (ca. 30 - 40 %ig).

Wir fanden nun ein deutlich besseres Verfahren, das diese Verbindungen überraschenderweise in wesentlich höherer Ausbeute und hoher Reinheit liefert.

Gegenstand der Erfindung ist daher ein Verfahren zur Herstellung der Verbindung der Formel I

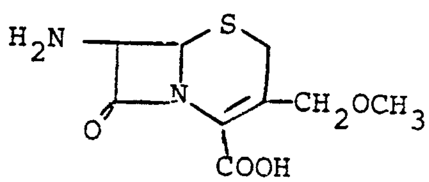

(I)

das dadurch gekennzeichnet ist, daß man 7-Amino-cephalosporansäure (7-ACS) der Formel II

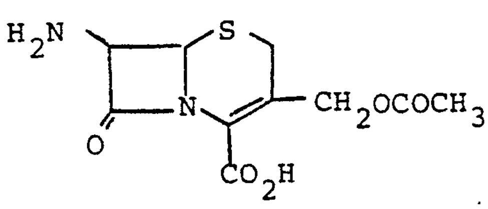

(II)

oder ein geeignetes Salz derselben, mit einem Gemisch aus Methansulfonsäure, Trifluormethansulfonsäure und Methanol umsetzt und nach beendeter Reaktion das Produkt der Formel I aus der Reaktionslösung ausfällt.

Die 7-ACS oder deren Salze können entweder als Pulver in das Reaktionsgemisch eingetragen oder in einem Teil des verwendeten Methanols suspendiert und so der Reaktionslösung zugeführt werden. Es ist auch möglich, die 7-ACS oder ihre Salze in eine Methansulfonsäure/Trifluormethansulfonsäure-Lösung einzutragen und anschließend das Methanol zuzusetzen.

Die verwendete Menge an Methansulfonsäure kann zwischen etwa 7 und 40, vorzugsweise zwischen 8 und 12, insbesondere bei 10 Äquivalenten, bezogen auf eingesetzte 7-ACS, liegen. Die Trifluormethansulfonsäuremenge kann etwa 0,5 bis 5, vorzugsweise 0,8 bis 1,5, insbesondere 1 Äquivalent, bezogen auf die 7-ACS, betragen. Die Methanol-Komponente kann in einer Menge von etwa 2 bis 20, vorzugsweise 4 bis 7, insbesondere von 5 Äquivalenten, auch wieder bezogen auf die eingesetzte 7-ACS, zugegeben werden.

Die Reaktionstemperatur sollte zwischen etwa -10° C und +40° C, vorzugsweise zwischen +5° C und +15° C, liegen.

Die Reaktionszeit kann je nach Reaktionstemperatur etwa 5 Minuten bis 3 Stunden, vorzugsweise 2 bis 2,5 Stunden betragen.

Geeignete Salze der 7-ACS sind solche, die stabil sind und mit ihrer Säurekomponente eine definierte stöchiometrische Zusammensetzung besitzen.

Als besonders geeignete Salze der 7-ACS kommen solche mit aromatischen Sulfonsäuren, wie z.B. mit Benzolsulfonsäure, vorzugsweise solche mit Alkylbenzolsulfonsäuren, wie z.B. mit p-Methyl- oder p-Ethylbenzolsulfonsäure, in Betracht. Besonders geeignet ist das 7-ACS-p-toluolsulfonatdihydrat, das in der DE-AS 15 45 898 beschrieben ist, sowie seine wasserfreie Form.

Die Isolierung der Verbindung der Formel I erfolgt nach Hinzufügen von Eiswasser durch Zugabe einer Base zu der hydrolysierten Reaktionsmischung und Einstellen des pH-Wertes auf etwa 2,5 bis 3,5. Als Basen eignen sich beispielsweise konz. Ammoniak, 10 - 40 %ige Kalilauge, vorzugsweise etwa 40 %ige Natronlauge. Das ausgefallene Produkt wird dann noch nachgewaschen, beispielsweise mit Wasser, Aceton und Ether, und in üblicher Weise getrocknet.

Das erfindungsgemäße Verfahren zeichnet sich gegenüber den bekannten Methoden dadurch aus, daß es die Verbindung der Formel I in besserer Ausbeute und höherer Reinheit liefert. Das Produkt enthält außerdem keine 7-ACS mehr und braucht nicht mehr weiter gereinigt zu werden.

Die folgenden Beispiele erläutern die vorliegende Erfindung, schränken sie jedoch nicht darauf ein.

**Ausführungsbeispiel 1**

7-Amino-3-methoxymethyl-ceph-3-em-4-carbonsäure

Zu 132,5 ml Methansulfonsäure (2,0 Mol) und 17,6 ml Trifluormethansulfonsäure (0,2 Mol) wurden bei -10° C bis +5° C innerhalb von 10 Minuten 20 ml Methanol zugetropft. Anschließend wurde innerhalb von 12 Minuten eine zuvor hergestellte Mischung aus 54,4 g 7-ACS (0,2 Mol) und 20 ml Methanol so eingetragen, daß die Temperatur zwischen 4 und 6° C blieb. Die anfänglich vorhandene Suspension ging allmählich in Lösung. Nach 130 Minuten wurde die Reaktionslösung auf 200 ml Eiswasser gegossen. Nach 2,5 Stunden Rühren wurde mit 40 %iger Natronlauge unter Kühlung (10 bis 20° C) auf pH = 2,5 gestellt, der sandartige Niederschlag abfiltriert, fünfmal mit Wasser und anschließend mit Aceton gewaschen und getrocknet. Man erhielt 27,6 g der gewünschten Titelverbindung mit einem Gehalt von 90 % lt. HPLC.

$^1$H-NMR (270 MHz, DMSO-$d_6$): $\delta$ = 3.20 (s, 3H, $OCH_3$), 3.35-3.60 (AB-System, 2H, -S-$CH_2$-), 4.15 (s, 2H, -$CH_2$-O-), 4.76 (d, J = 4 Hz, 1H, 6-H), 4.98 (d, J = 4 Hz, 1H, C-7).

IR (KBr): 1800 $cm^{-1}$ ($\beta$-Lactam-Carbonyl)

| | | C | H | N | S |
|---|---|---|---|---|---|
| $C_9H_{12}N_2O_4S$ | ber. | 44,25 | 4,95 | 11,47 | 13,13 |
| (244,265) | gef. | 44,0 | 5,0 | 11,4 | 13,5 |

**Ausführungsbeispiel 2**

Zu einer Lösung von 32,5 ml (0,5 Mol) Methansulfonsäure und 4,4 ml (0,05 Mol) Trifluormethansulfonsäure wurden bei 2 - 5° C 10 ml Methanol und anschließend 24 g (0,054 Mol) wasserfreies 7-ACS-p-toluolsulfonat innerhalb von 10 Minuten eingetragen. Die anfängliche Suspension löste sich rasch auf und die erhaltene Lösung wurde nach insgesamt 130 Minuten Rühren bei 2 - 5° C mit 50 ml Eiswasser versetzt. Nach 2,5 Stunden Rühren bei 20° C wurde unter Eiskühlung mit ca. 40 ml 40 %iger Natronlauge der pH-Wert auf 2,5 gestellt. Der ausgefallene Niederschlag wurde anschließend abgesaugt und noch mit Eiswasser, Aceton und Diethylether gewaschen. Nach dem Trocknen wurden 7,2 g der gewünschten Titelverbindung erhalten, die in ihren physikalischen und chemischen Eigenschaften mit denen des Ausführungsbeispiels 1 identisch war und lt. HPLC einen Gehalt von mindestens 90 % hatte.

**Patentansprüche**

1. Verfahren zur Herstellung der Verbindung der Formel I

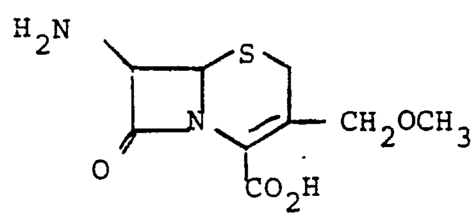

(I)

dadurch gekennzeichnet, daß man die Verbindung der Formel II,

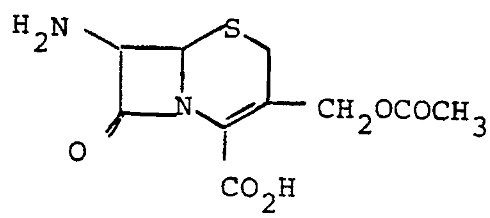

(II)

oder ein geeignetes Salz desselben mit einem Gemisch aus Methanol, Methansulfonsäure und Trifluormethansulfonsäure umsetzt.

2. Verfahren nach Anspruch 1, dadurch gekennzeichnet, daß als geeignetes Salz ein solches mit einer aromatischen Sulfonsäure verwendet wird.

3. Verfahren nach Ansprüchen 1 und 2, dadurch gekennzeichnet, daß als Salz das p-Toluolsulfonatdihydrat der Verbindung II oder seine wasserfreie Form verwendet wird.

4. Verfahren nach Ansprüchen 1 bis 3, dadurch gekennzeichnet, daß die Reaktion bei einem Verhältnis der Komponenten zur 7-ACS von etwa 7 bis 40 Äquivalenten Methansulfonsäure, etwa 0,5 bis 5 Äquivalenten Trifluormethansulfonsäure und etwa 2 bis 20 Äquivalenten Methanol durchgeführt wird.

5. Verwendung der nach den Ansprüchen 1 bis 4 erhaltenen Verbindung der Formel I zur Herstellung eines Cephalosporin-Antibiotikums durch Acylierung der Aminogruppe.